# EUROPÄISCHE PATENTSCHRIFT

(11) **EP 2 740 704 B1**
(45) Veröffentlichungstag und Bekanntmachung des Hinweises auf die Patenterteilung: **02.09.2015**
(21) Anmeldenummer: 14158068.8
(22) Anmeldetag: 24.01.2012
(51) Int. Cl.: B66C 15/00, B66C 13/16, B66D 1/54, G01N 3/08, G01N 33/36, B66C 15/06

(54) **Vorrichtung zur Erkennung der Ablegereife eines hochfesten Faserseils beim Einsatz an Hebezeugen**
Device for detecting the discarding state of a high-strength fibre rope during use on a lifting gear
Dispositif de détection de l'état d'usure d'un câble en fibre haute résistance pour une utilisation sur des engins de levage

(30) Priorität: 24.01.2011 DE 202011001846 U
(43) Veröffentlichungstag der Anmeldung: 11.06.2014
(62) Teilanmeldung aus: 12701226.8
(73) Patentinhaber: Liebherr-Components Biberach GmbH, 88400 Biberach/Riß (DE)
(72) Erfinder: Ilaka, Mupende, 89231 Neu-Ulm (DE); Zerza, Horst, 88400 Biberach (DE)
(74) Vertreter: Thoma, Michael

(56) Entgegenhaltungen:
- DE-A1- 19 956 265
- JP-A- 9 012 271
- JP-A- 9 188 496
- JP-A- 10 318 741
- JP-A- S60 253 810
- JP-A- 2000 327 272
- JP-A- 2001 192 183
- JP-A- 2005 189 157
- US-A- 5 834 942
- US-A1- 2003 052 695
- US-B2- 7 117 981

## Beschreibung

Die vorliegende Erfindung betrifft allgemein Hebezeuge wie Krane, die anstelle von Stahlseilen hochfeste Faserseile verwenden. Die Erfindung betrifft dabei insbesondere eine Vorrichtung zur Erkennung der Ablegereife eines hochfesten Faserseils beim Einsatz an solchen Hebezeugen, mit einer Erfassungseinrichtung zur Erfassung zumindest einer Seilkenngröße sowie einer Auswerteeinheit zur Auswertung der Seilkenngröße sowie Bereitstellung eines Ablegesignals in Abhängigkeit der Seilkenngrößenauswertung.

Aus der Schrift US2003/0052695 ist es bekannt, Aramid-Faserseile auf ihre Rest-Festigkeit und Ablegereife durch eine Schallwellenmessung zu überprüfen, bei der das Seil mit Schallwellen beaufschlagt, die Laufzeit der Schallwellen durch das Seil gemessen und aus der gemessenen Laufzeit die Rest-Festigkeit bestimmt wird. Aus der Schrift US 711798 ist es ferner bekannt, einen flachen Gurt mit Markierungen zu versehen und eine Dehnung des Gurts anhand von Abstandsveränderungen der Markierungen zu überwachen.

In jüngerer Zeit wird an Kranen versucht, anstelle der bewährten und seit vielen Jahren eingesetzten Stahlseile hochfeste Faserseile aus Kunstfasern wie beispielsweise Aramidfasern (HMPA), Aramid-/Kohlefasergemischen, hochmodulare Polyethylen-Fasern (HMPE) oder Poly(p-phenylene-2,6-benzobisoxazole)-Fasern (PBO) zu verwenden. Der Vorteil solcher hochfesten Faserseile liegt in ihrem geringen Gewicht. Bei gleichen Seildurchmessern und gleichen oder höheren Zugfestigkeiten sind solche hochfesten Faserseile deutlich leichter als entsprechende Stahlseile. Insbesondere bei hohen Kranen mit entsprechend großen Seillängen kommt hierdurch eine größere Gewichtsersparnis zustande, die in die Eigenlast des Krans eingeht und zu entsprechend höheren Nutzlasten bei ansonsten unveränderter Bauart des Krans führt.

Eine nachteilige Eigenschaft derartiger hochfester Faserseile ist jedoch ihr Bruchverhalten bzw. ihr Versagen ohne deutliche, längere Vorankündigung. Während sich bei Stahlseilen der Verschleiß deutlich zeigt und ein Versagen über längere Zeit vorher ankündigt, beispielsweise durch den Bruch einzelner Stahldrähte und ein entsprechendes Aufspleißen, das einfach bemerkt wird, zeigen hochfeste Faserseile kaum Anzeichen an übermäßigem Verschleiß, die mit dem Auge einfach wahrnehmbar wären und sich über längere Zeit vor dem eigentlichen Versagen deutlich zeigen würden. Insofern bedarf es intelligenter Überwachungsmaßnahmen, um die Ablegereife von hochfesten Faserseilen rechtzeitig zu erkennen.

Aus der DE 199 56 265 B4 ist eine Vorrichtung zur Überwachung des Betriebs von Hubwinden an Kranen bekannt, die die Seilkraft des Hubseils und den Hebelarm des Hubseils auf die Seilwinde überwacht und hieraus die auf die Seilwinde wirkenden Lastspiele bestimmt, die in einem Lastkollektivzähler abgelegt werden. Dieser Lastkollektivzähler ist in die Hubwinde integriert, um bei Aus- und Wiedereinbau der Hubwinde deren Historie nachvollziehbar zu bewahren. Weiterhin ist aus der EP 0 749 934 A2 ein Lastkollektivzähler bekannt, der die auftretenden Lastwechsel bestimmt, zu jedem Lastwechsel die die Hubwinde beanspruchende Seilkraft bestimmt, hieraus das Lastkollektiv berechnet und unter Einbeziehung der sog. Wöhlerlinien die Restlebensdauer der Hubwinde berechnet und anzeigt.

Derartige Überwachungsmaßnahmen der Hubwinde können jedoch die Restlebensdauer bzw. die Ablegereife eines hochfesten Faserseils nicht wirklich verlässlich angeben, da die hochfesten Faserseile vielerlei Verschleiß beeinflussenden Belastungen und Beeinträchtigungen unterliegen, die unabhängig von der Windenbeanspruchung sind, so z.B. die Umlenk- und Biegebeanspruchungen an Umlenkrollen, externe Stöße und Schläge auf das Seil, Oberflächenverunreinigungen von das Seil berührenden Bauteilen etc. Andererseits sind starre Lebenszeitvorgaben für hochfeste Faserseile hinsichtlich wirtschaftlicher Ausnutzung der tatsächlichen Lebenszeit und Einhaltung der notwendigen Sicherheit kaum miteinander kompatibel, da in Abhängigkeit der Einsatzbedingungen und der äußeren Einwirkungen auf das hochfeste Faserseil dessen Lebensdauer und Verschleiß stark schwanken kann.

Hiervon ausgehend liegt der vorliegenden Erfindung die Aufgabe zugrunde, eine verbesserte Vorrichtung zur Bestimmung der Ablegereife von hochfesten Faserseilen anzugeben, die Nachteile des Standes der Technik vermeidet und Letzteren in vorteilhafter Weise weiterbildet. Vorzugsweise soll eine verlässliche, präzise Bestimmung der Ablegereife erzielt werden, die die Restlebensdauer des Faserseils wirtschaftlich ausnutzt, ohne die Sicherheit zu gefährden und hierfür mit einfachen, auch unter schweren Einsatzbedingungen für Baumaschinen verlässlich arbeitenden Erfassungseinrichtungen auskommt.

Erfindungsgemäß wird diese Aufgabe durch eine Vorrichtung nach Anspruch 1 gelöst. Bevorzugte Ausgestaltungen der Erfindung sind Gegenstand der abhängigen Ansprüche.

Nach einem vorteilhaften Aspekt der vorliegenden Erfindung wird eine Längung des Faserseils überwacht und zur Bestimmung der Ablegereife herangezogen. Die Überwachung der Längung des Faserseils geht von der Überlegung aus, dass ein zunehmender Verschleiß bzw. Schaden am Faserseil bzw. die Annäherung an die Ablegereife mit einer Längung des Faserseils gegenüber dessen Ursprungszustand einhergeht, so dass die Überwachung der Längung des Faserseils als Indikator für die Ablegereife genutzt werden kann. Die Erfassungseinrichtung kann hierzu Bestimmungsmittel zur Bestimmung der Längung des Faserseils aufweisen, wobei die Auswerteeinheit die bestimmte Längung mit einer zulässigen maximalen Längung abgleicht. Sobald die Längung ein vorbestimmtes Maß überschreitet, kann die Ablegereife angezeigt werden.

Bei der Bestimmung der Längung kann hierbei verschieden vorgegangen werden. Insbesondere kann in einem ersten Betriebsmodus die Längung des im Wesentlichen gesamten unter Belastung stehenden Seils bzw. Seilabschnitts bestimmt und überwacht werden. Alternativ oder zusätzlich kann in einem zweiten Betriebsmodus die Längung des Faserseils abschnittsweise überprüft werden dahingehend, ob und inwieweit sich vorbestimmte Abschnitte des Faserseils längen.

Nach einer vorteilhaften Ausführung der Erfindung können die Bestimmungsmittel zur Bestimmung der Längung einen Positionssensor zur Erfassung der Position eines vorbestimmten Seilabschnitts sowie einen Seilwindenstellungssensor zur Erfassung der beim Anfahren der vorbestimmten Seilposition sich einstellenden Windenstellung aufweisen. Der genannte Positionssensor kann beispielsweise erfassen, wenn ein oberer Abschaltpunkt für den Lasthaken erreicht wird und/oder wenn ein am Seil angebrachter Signalgeber beispielsweise in Form einer Markierung eine vorbestimmte Stelle entlang des Seilwegs erreicht. Der Seilwindenstellungssensor erfasst die in diesem Moment bzw. bei Erreichen der genannten Position herrschenden Seilwindenstellung, so dass die Auswerteeinheit die Seillängung aus einer Veränderung der sich einstellenden Windenstellung bestimmen kann. Weicht die Windenstellung bei Erreichen der vorbestimmten Position des vorbestimmten Seilpunkts zu weit von einer Sollstellung ab, kann die Ablegereife angenommen bzw. ein Ablegesignal abgegeben werden.

Alternativ oder zusätzlich kann das Faserseil über seine Länge verteilt mit mehreren Signalgebern beispielsweise in Form von Markierungen, Transpondern, Signalreflektoren oder dergleichen versehen und somit in mehrere Längenabschnitte unterteilt sein. Die Bestimmungsmittel zur Bestimmung der Seillängung bestimmen den Abstand zwischen jeweils zwei Signalgebern, woraus die Auswerteeinheit die Längung der entsprechenden Seilabschnitte bestimmen und auf Veränderungen hin überwachen kann. Treten in einem oder mehreren Seilabschnitten Längungen auf, die einzeln oder in ihrer Summe betrachtet über einen jeweiligen Grenzwert für die zulässige Längung hinausgehen, kann die Auswerteeinheit ein Ablegesignal abgeben.

In Weiterbildung der Erfindung kann die genannte Erfassungseinrichtung hierbei derart ausgebildet sein, dass eine beispielsweise elektronische Messeinrichtung an einem bestimmten Punkt entlang des Seilwegs das Vorbeikommen bzw. Auftreten des genannten Signalgebers erfasst und den Längenabstand bis zum nächsten Signalgeber bei vorzugsweise konstanter Seilgeschwindigkeit misst. Hierdurch kann die Seillänge auf beliebige Messpunkte und in beliebige Seilabschnitte aufgeteilt bzw. unterteilt werden, so dass der Dehnungsverlauf des Seiles über die gesamte Seillänge bestimmt werden kann und in der Auswerteeinrichtung ausgewertet werden kann, in welchem Seilabschnitt der Grenzwert erreicht wurde und das Seil ausgetauscht werden muss, oder, wenn möglich, um den Ablegebereich, d.h. den überdehnte Seilbereich gekürzt werden muss.

Vorteilhafterweise wird die Prüfung auf Längung unter vorgegebenen Randbedingungen, insbesondere vorbestimmter Seillast, beispielsweise durch Anhängen einer Prüflast durchgeführt, um eine Varianz der Prüfergebnisse aufgrund variierender Randbedingungen zu eliminieren.

Es wird nach einem vorteilhaften Aspekt der vorliegenden Erfindung vorgeschlagen, sich bei der Bestimmung der Ablegereife nicht auf ein einziges Kriterium zu verlassen, sondern die Problematik der nur schwer erfassbaren Vorankündigungszeichen dadurch zu umgehen, dass verschiedene relevante Kenngrößen des Faserseils auf Veränderungen hin überwacht werden und die Ablegereife bei einer größeren Veränderung einer einzelnen Kenngröße oder mehreren kleineren Veränderungen mehrerer Kenngrößen bestimmt wird. Insbesondere umfaßt die Erfassungseinrichtung der Vorrichtung zur Erkennung der Ablegereife mehrere, verschieden ausgebildete Erfassungsmittel zur magnetischen, mechanischen, optischen und/oder elektronischen Erfassung mehrerer verschiedener Seilkenngrößen, die von der Auswerteeinheit einzeln und/oder in Kombination miteinander zur Erkennung der Ablegereife auswertbar sind. Der Heranziehung verschiedener Seilkenngrößen, wie beispielsweise die genannte Querdrucksteifigkeit und Querschnittsveränderung oder alternativ oder zusätzlich hierzu eine Seillängung und magnetische Seileigenschaften oder anderer mechanischer, optischer und/oder elektronischer Seilkenngrößen, für die Bestimmung der Ablegereife liegt die Überlegung zugrunde, dass je nach Belastung und Einwirkungen auf das Faserseil es von Fall zu Fall eine andere Kenngröße sein kann, die den Seilverschleiß anzeigt bzw. die Ablegereife ankündigt, bzw. sich die Ablegereife ggf. auch nicht durch eine tatsächlich größere Veränderung einer nur einzigen Kenngröße, sondern durch kleinere Veränderungen mehrerer Kenngrößen zeigt.

In Weiterbildung der Erfindung ist die genannte Auswerteeinheit derart ausgebildet, dass ein Ablegesignal dann bereitgestellt wird, wenn zumindest eine der erfassten Seilkenngrößen bzw. deren Veränderung einen zugehörigen Grenzwert überschreitet/unterschreitet, sowie dann, wenn eine aus allen erfassten bzw. einer Untergruppe der erfassten Seilkenngrößen abgeleitete, mittelbare Seilkenngröße bzw. deren Veränderung einen zugehörigen Grenzwert überschreitet/unterschreitet.

In Weiterbildung der Erfindung ist die genannte Auswerteeinheit derart ausgebildet, dass nicht nur mehrere Seilkenngrößen per se erfasst und auf jeweilige Veränderungen untersucht bzw. mit Grenzwerten verglichen werden, sondern auch Abhängigkeiten zwischen den mehreren Seilkenngrößen berücksichtigt werden. Beispielsweise können zulässige Veränderungen und/oder zulässige Grenzwerte für eine Seilkenngröße verschoben bzw. verändert werden, wenn eine andere Seilkenngröße eine vorbestimmte Veränderung erfahren hat. Hierdurch können insbesondere komplexere Ermüdungen bzw. Schädigungen erfasst und die Ablegereife erkannt werden. Geht man beispielsweise davon aus, dass ein Anstieg der Querdrucksteifigkeit mit einer Abnahme des Seildurchmessers einhergeht, kann bei Erfassung einer angestiegenen Querdrucksteifigkeit von der Auswerteeinheit der Grenzwert für den Seildurchmesser abgesenkt und/oder der Sollbereich für den zulässigen Seildurchmesser verkleinert werden. Stellt dann die Messung fest, dass der Seildurchmesser den abgesenkten Seildurchmessergrenzwert unterschreitet und/oder aus dem verkleinerten Sollbereich herausfällt, kann ein Ablegesignal abgegeben werden. In ähnlicher Weise können alternativ oder zusätzlich hierzu weitere Abhängigkeiten zwischen den verschiedenen Seilkenngrößen von der Auswerteeinheit berücksichtigt werden, beispielsweise die vorgenannte Abhängigkeit zwischen Seilsteifigkeit und Seillängung, beispielsweise dergestalt, dass bei einer höheren Biegesteifigkeit eine zunehmende Seillänge erwartet und durch entsprechende Grenzwerte berücksichtigt wird.

Hierbei können in Weiterbildung der Erfindung verschiedene Seilkenngrößen herangezogen werden. Gemäß einem weiteren Aspekt der vorliegenden Erfindung wird vorgeschlagen, eine Veränderung der Querdrucksteifigkeit bzw. des Seilquerschnitts zu überwachen und als Indikator für die Ablegereife zu nutzen. Insbesondere kann die Erfassungseinrichtung zur Erfassung von Seilveränderungen Querdrucksteifigkeits- und/oder Querschnitts-Bestimmungsmittel zur Bestimmung der Querdrucksteifigkeit bzw. des Seilquerschnitts aufweisen, wobei die Auswerteeinheit die Querdrucksteifigkeit bzw. den bestimmten Seilquerschnitt auf Veränderungen hin überwacht und ggf. ein Ablegesignal bereitstellt.

Bei Dauerversuchen von hochfesten Faserseilen kann gezeigt werden, dass mit steigender Belastung und Biegewechselzahl die Querdrucksteifigkeit sich in charakteristischer Weise verändert, insbesondere eine Zunahme zeigt. Das Maß der sich verändernden Querdrucksteifigkeit kann daher in vorteilhafter Weise zur Festlegung des Zeitpunktes der Ablegereife mit herangezogen werden. Dabei kann ein Anstieg der Querdrucksteifigkeit mit einer Abnahme des Seildurchmessers einhergehen. Das Seil kann eine höhere Biegesteifigkeit erhalten und/oder es kann eine bleibende, messbare Seillängung eintreten. Insbesondere kann eine Abhängigkeit der Veränderung der Querdrucksteifigkeit zur Veränderung des Seildurchmessers erfasst werden, wobei insbesondere eine Zunahme der Querdrucksteifigkeit in Abhängigkeit zum kleiner werdenden Seildurchmesser erfasst werden kann. Alternativ oder zusätzlich kann eine Abhängigkeit der Veränderung der Seilsteifigkeit von der Veränderung der Seillänge bestimmt werden, wobei insbesondere eine Zunahme der Seilsteifigkeit in Abhängigkeit einer Zunahme der Seillänge bestimmt werden kann. Die Ausgabe des Ablegesignals kann dabei grundsätzlich alleine in Abhängigkeit der überwachten Querdrucksteifigkeit bzw. des Seilquerschnitts erfolgen. Durch die Berücksichtigung der Abhängigkeiten der verschiedenen Seilkenngrößen voneinander kann allerdings vorteilhafterweise eine präzisere Bestimmung der Ablegereife erfolgen.

Die Querdrucksteifigkeit des Seiles kann hierbei grundsätzlich in verschiedener Art und Weise bestimmt werden. In vorteilhafter Weiterbildung der Erfindung können der Messung hierbei die errechneten Daten aus Trommeldurchmesser, Seildurchmesser, Seilspezifikation und Zugkraft zugrunde liegen bzw. kann der Messvorgang in Abhängigkeit der genannten errechneten Daten aus Trommeldurchmesser, Seildurchmesser, Seilspezifikation und Zugkraft gesteuert werden und die entsprechenden Messparameter eingestellt werden. Insbesondere kann die Querdrucksteifigkeit unter Beaufschlagung des Seiles mit einer vorbestimmten Zugbelastung durchgeführt werden, wobei vorteilhafterweise die vorbestimmte Zugbelastung im Bereich tatsächlich auftretender Zuglasten im bestimmungsgemäßen Betrieb des Hubzeugs gewählt werden kann. Durch die Bestimmung der Querdrucksteifigkeit unter Zugbelastung des Seils können Schädigungen bzw. Ermüdungserscheinungen des Seils besser bestimmt werden.

In Weiterbildung der Erfindung kann zur Bestimmung der Querdrucksteifigkeit ein Seilabschnitt oder mehrere Seilabschnitte mit jeweils einer vorbestimmten Querkraft beaufschlagt, insbesondere geklemmt werden, wobei die sich unter der Querkraft einstellende Veränderung des Durchmessers und/oder Veränderung des Querschnitts des Seils erfasst bzw. bestimmt wird. Dabei kann bei vorgegebener Querkraft die sich einstellende Querschnitts- bzw. Durchmesserveränderung gemessen und/oder alternativ die zur Erreichung einer vorbestimmten Querschnitts- und/oder Durchmesserveränderung erforderliche Querkraft gemessen werden. Alternativ oder zusätzlich können variierende Querkräfte aufgebracht und die sich in Abhängigkeit der variierenden Querkraft einstellende Querschnitts- bzw. Durchmesserveränderung bestimmt werden, und/oder alternativ die für das Erreichen verschiedener Querschnitts- bzw. Durchmesserveränderungen benötigten Querkräfte gemessen werden.

In vorteilhafter Weiterbildung der Erfindung kann das Seil zur Bestimmung seiner Querdrucksteifigkeit zwischen zwei einander gegenüberliegende Klemmbacken, die vorteilhafterweise jeweils eine Seilrille besitzen können, eingebracht und von den Klemmbacken geklemmt werden, indem diese durch eine geeignete Stellvorrichtung aufeinander zu bewegt werden.

Der Seilquerschnitt kann hierbei grundsätzlich in verschiedener Art und Weise erfasst werden. Vorteilhafterweise können die genannten Seilquerschnittsbestimmungsmittel Durchmessererfassungsmittel zur Erfassung des Seildurchmessers in zumindest zwei verschiedenen Ebenen umfassen und die Seilquerschnittsfläche aus den genannten zwei bestimmten Seildurchmessern bestimmen. Eine solche Erfassung eines Seilabschnitts oder mehrerer Seilabschnitte in mehreren Ebenen kann auch bei der vorgenannten Bestimmung der Querdrucksteifigkeit vorgesehen werden, beispielsweise dergestalt, dass der jeweilige Seilabschnitt gleichzeitig oder nacheinander von mehreren Klemmbackenpaaren, die in verschiedenen Ebenen einander zugeordnet positioniert sind, geklemmt wird. Grundsätzlich wäre es zwar auch denkbar, die Seilquerschnittsfläche aus nur einem Seildurchmesser, der in einer Ebene bestimmt wurde, zu ermitteln bzw. abzuleiten. Vorteilhafterweise jedoch wird der Seilquerschnitt bzw. die Seilquerschnittsfläche aus zwei Seildurchmessern bestimmt, die in verschiedenen, näherungsweise senkrecht zueinander stehenden Ebenen bestimmt wurden, da hierdurch für die Festigkeit des Faserseils unschädliche Querschnittsveränderungen bzw. -verformungen berücksichtigt und vorschnelle Verschleißannahmen vermieden werden können. Hochfeste Faserseile zeigen unter Querbelastungen, wie sie beispielsweise an Seilrollen oder an der Seilwinde auftreten können, ovalisierende Querschnittsveränderungen, d.h. der im Ausgangszustand an sich kreisrunde Querschnitt verändert sich zu einem flachgedrückten Profil hin, was an sich noch nicht schädlich für die Haltbarkeit bzw. Festigkeit des Faserseils ist. Verändert sich der Seilquerschnitt jedoch derart, dass die Querschnittsfläche abnimmt, wird dies vorteilhafterweise als ein Anzeichen für einsetzenden Verschleiß angesehen. Insbesondere kann die Auswerteeinrichtung ein Ablegesignal bereitstellen, wenn der Seilquerschnitt eine vorbestimmte Verjüngung zeigt bzw. eine Verkleinerung der Seilquerschnittsfläche ein vorbestimmtes Maß überschreitet.

Die Durchmesserbestimmung kann hierbei in verschiedener Art und Weise erfolgen. Beispielsweise könnte eine optische Abtastung mittels einer Lichtbestrahlung und einem zugeordneten Sensor zur Erfassung der Schattenbreite vorgesehen sein. In vorteilhafter Weiterbildung der Erfindung jedoch erfolgt eine mechanische Abtastung des Seils von gegenüberliegenden Seiten her, um den Seildurchmesser zu bestimmen. Vorzugsweise kann zumindest ein elastisch vorspannbares Klemmmittelpaar, vorzugsweise in Form von gegen das Seil drückbaren Seilrollen oder Klemmbacken mit Seilrillen vorgesehen sein, denen ein Abstandsmesser zugeordnet ist, um den Abstand der Klemmmittel voneinander im an das Seil angelegten Zustand zu messen.

In vorteilhafter Weiterbildung der Erfindung können die Querdrucksteifigkeitsbestimmung und die Seilquerschnittsbestimmung bzw. Durchmesserbestimmung durch dasselbe Klemmmittelpaar bzw. dieselben Klemmmittelpaare durchgeführt werden, so dass Messzeiten gespart und verschiedene Einspannungen vermieden werden können. Beispielsweise kann eine reine Durchmesser- bzw. Querschnittsbestimmung unter ausreichend kleinen Querandruckkräften erfolgen, die sodann erhöht werden können, um die Messung der Querdrucksteifigkeit durchzuführen.

Um die Durchmesserbestimmung nicht durch Auslenkungen des Seils zu beeinträchtigen, können die genannten Abtastmittel beweglich aufgehängt sein, so dass sie im an das Seil angelegten Zustand Seilbewegungen, insbesondere Seilquerbewegungen, mitmachen können. Insbesondere können die vorgenannten vorspannbaren Klemmmittel in Form der Seilrollen einerseits relativ zueinander sowie andererseits gemeinsam quer und/oder parallel zur Seillängsrichtung bewegt werden, um auch bei ungewollten Seilauslenkungen exakt den Seildurchmesser bestimmen zu können.

Vorteilhafterweise erfolgt die Seilmessung in mindestens zwei Ebenen, um Abweichungen des Seilquerschnitts von der Kreisform bei der Bestimmung der Querschnittsfläche eliminieren zu können. Hierfür können beispielsweise zwei Seilrollenpaare oder Klemmbacken vorgesehen sein, die in zueinander senkrechten Ebenen angeordnet sind und jeweils elastisch gegeneinander spannbar sind.

In Weiterbildung der Erfindung können alternativ oder zusätzlich zu der genannten Querdrucksteifigkeit bzw. Querschnittsfläche oder -form verschiedene andere Seilkenngrößen herangezogen werden Nach einem weiteren Aspekt der vorliegenden Erfindung wird hierbei in vorteilhafter Weise eine Veränderung eines in das Faserseil eingebetteten Indikatorprofils, das aus einem anderen Material als die Seilfasern besteht, überwacht. Mittels eines derartigen Indikatorprofils, das im Kern der Litze eingebettet oder auch zwischen den Fasersträngen des Faserseils angeordnet sein kann, kann die nur schwer erfassbare Veränderung der Fasern bzw. Faserstränge des Faserseils selbst umgangen werden, insbesondere wenn das Indikatorprofil hinsichtlich seiner Ausbildung und/oder hinsichtlich seines Materials so gewählt wird, dass das Indikatorprofil schneller als die Faserstränge des Faserseils Veränderungen zeigt und/oder solche Veränderungen leichter erfassbar sind. Die Überwachung eines solchen Indikatorprofils im Faserseil kann hierbei auch nur für sich ohne Überwachung weiterer Kenngrößen besondere Vorteile mit sich bringen.

Insbesondere kann das Indikatorprofil aus einem ein Magnetfeld beeinflussenden und/oder magnetisch leitenden und/oder magnetisierbaren Material, vorzugsweise aus einem metallischen Endlosprofil, bestehen. Die Erfassungsmittel sind dabei vorteilhafterweise magnetisch arbeitend ausgebildet, wobei insbesondere ein Magnetfeldsensor vorgesehen sein kann, mittels dessen die magnetischen Eigenschaften des genannten Indikatorprofils bestimmt werden können. Insbesondere verändern sich die magnetischen Eigenschaften des Indikatorprofils bei einem Bruch des Indikatorprofils, so dass eine entsprechende Veränderung des Magnetflusses bzw. des Magnetfelds leicht erfasst und als Verschleißanzeige genutzt werden kann. Tritt ein Bruch des magnetisch leitenden Indikatorprofils auf, kann dies durch eine magnetinduktive Überwachung erkannt werden bzw. eine entsprechende Unterbrechung des Magnetfelds erfasst werden.

Alternativ oder zusätzlich zu einer solchen magnetisch arbeitenden Ausbildung des Indikatorprofils und der zugehörigen Erfassungsmittel könnten Veränderungen des genannten Indikatorprofils ggf. auch anders überwacht und auf andere Überwachungsprinzipien zurückgegriffen werden. Beispielsweise könnte das Indikatorprofil elektrisch leitend ausgebildet und mit entsprechend ausgebildeten Erfassungsmitteln die elektrische Leitfähigkeit des Faserseils bzw. des darin vorgesehenen Indikatorprofils überwacht werden. Alternativ oder zusätzlich könnte auch eine thermische Leitfähigkeit des genannten Indikatorprofils überwacht werden, wobei hier vorteilhafterweise das Indikatorprofil aus einem thermisch gut leitenden Material, beispielsweise aus einem Silberdraht ausgebildet ist.

Vorteilhafterweise ist das genannte Indikatorprofil, das in dem Faserseil eingebettet ist und aus einem anderen Material als die Seilfasern besteht, hinsichtlich seiner Widerstandsfähigkeit gegen Seilspannungen, Dehnung, Zug, Biegung, Torsion, UV-Strahlung, Wasseraufnahme und/oder Temperatur schwächer als das Faserseil ausgebildet derart, dass das Indikatorprofil deutlich schneller versagt als das Faserseil bzw. dessen Faserstränge. Hierdurch ist sichergestellt, dass rechtzeitig eine Veränderung des Indikatorprofils feststellbar ist, bevor ein Versagen des Faserseils auftritt. Ein Bruch des genannten Indikatorprofils hat noch keine nennenswerte Auswirkung auf die Festigkeit des Faserseils selbst, kann jedoch leicht bestimmt werden und rechtzeitig vor dem Auftreten des Versagens des Seils erfasst werden.

In Weiterbildung der Erfindung überwacht die Erfassungseinrichtung, in welchem Seilabschnitt eine Veränderung des Seils, die zur Bestimmung der Ablegereife herangezogen wird, auftritt, um den verschlissenen bzw. schadhaften Seilabschnitt identifizieren und ggf. das restliche Seil noch weiter benutzen zu können, beispielsweise indem der schadhafte Teil abgetrennt wird. In Weiterbildung der Erfindung können den vorgenannten Erfassungsmitteln Seilweg- und/oder - positionserfassungsmittel zugeordnet sein, die den zurückgelegten Seilweg bzw. die Position des auf Veränderungen hin überwachten Seilabschnitts bestimmen. Insbesondere können die genannten Seilweg- bzw. -positionserfassungsmittel eine Seilwindenstellung oder -position erfassen, die gegeben ist, wenn der auf Veränderungen hin zu untersuchende Seilabschnitt gerade im Bereich der entsprechenden Erfassungseinrichtung ist und auf Veränderungen hin tatsächlich überwacht wird. Aus der genannten Seilwindenstellung kann dann in der Auswerteeinrichtung rückgerechnet werden, welcher Seilabschnitt schadhaft bzw. verschlissen ist.

Nach einer vorteilhaften Weiterbildung der Erfindung kann für die Bestimmung der Ablegereife des Faserseils auch das auf das Seil wirkende Lastkollektiv herangezogen werden, insbesondere die auf das Seil wirkenden Zugbeanspruchungen und die auf das Seil einwirkenden Biegewechsel. Hierzu kann ein Lastkollektivzähler vorgesehen sein, der als auf das Faserseil einwirkende Lastkollektiv zumindest die Seilzugbelastung und die Biegewechselanzahl erfasst. Die Ermittlung und Auswertung der genannten Messdaten ist über entsprechende Bestimmungsmittel bzw. Erfassungsmittel oder Sensoren möglich, deren Messdaten in der Auswerteeinrichtung verarbeitet und ausgewertet werden. Insbesondere kann ein Lastsensor die laufende Belastung des Seils über die Betriebszeit des Seils erfassen. Zur Bestimmung der Biegewechsel kann ein Drehwegsensor auf der Trommel der Seilwinde die Seillänge bestimmen, die beansprucht wird. In der Auswerteeinrichtung können die Lastdaten und die Seilweg- bzw. Biegewechseldaten miteinander verknüpft werden, um ein Lastkollektiv zu bestimmen, das mit einem vorbestimmten, zulässigen maximalen Lastkollektiv verglichen werden kann. Wird die Anzahl des maximal zulässigen Lastkollektivs erreicht, kann die Auswerteeinheit ein entsprechendes Ablegesignal ausgeben.

Bei der rechnerischen Bestimmung der auf das Seil wirkenden Lastkollektive kann grundsätzlich auf verschiedene analytische Ansätze zurückgegriffen werden. Hierbei kann von der Überlegung ausgegangen werden, aufgrund einer rechnerischen Akkumulation von Schädigungen bei verschiedenen Lastkollektiven auf unterschiedliche Schädigungsgrade zu schließen und diese im Steuerungssystem zu hinterlegen. Hierdurch kann bei einer bestimmten Vorgabe an Lastwechseln rechnerisch auf die hierdurch entstehenden Seilschädigungen geschlossen werden, wobei ein Grenzwert festgelegt werden kann, welcher eine Einschätzung der Ablegereife zulässt.

Beispielsweise kann bei der Auswertung der auftretenden Lastkollektive ein Zählverfahren eingesetzt werden, wobei beispielsweise die Amplitude der auftretenden Lasten über deren Summenhäufigkeit dargestellt werden kann. Da im Normalfall das Faserseil nicht nur einer immer wiederkehrenden, gleichen Belastung mit konstanter Amplitude, sondern einer in ihrer Höhe veränderlichen Belastung unterliegt, kann das sich in der Praxis ergebende Belastungskollektiv beispielsweise in einzelne Rechteckkollektive mit jeweils konstanter Belastung und einer Teilbelastungsspielzahl unterteilt bzw. getreppt werden. Beispielsweise nach dem an sich bekannten Verfahren der linearen Schadenakkumulation kann hierbei nun für jedes Teilkollektiv eine Teilschädigung berechnet werden, indem die Teilbelastungsspielzahl durch die maximal ertragbare Belastungsspielzahl geteilt wird. Die sich so ergebenden Teilschädigungen aller Teilkollektive können aufsummiert und als Angabe der Gesamtschädigung des Faserseils verwendet werden. In an sich ebenfalls bekannter Weise kann dieser Ansatz der linearen Schadensakkumulation auch in verschiedener Weise modifiziert werden, beispielsweise dahingehend, dass Teilkollektive, deren Lastamplituden unterhalb der Dauerfestigkeitsgrenze liegen nicht oder nur begrenzt berücksichtigt werden.

In Weiterbildung der Erfindung kann die Überwachung der vorgenannten Veränderungen des Faserseils, insbesondere die magnetische Veränderung eines Indikatorprofils, die Veränderung der Seillängung und/oder die Veränderung des Seildurchmessers durch Abgleich der entsprechend erfassten bzw. bestimmten Kenngrößen mit zuvor erfassten bzw. bestimmten Kenngrößen erfolgen. Insbesondere können die entsprechenden Referenzwerte für die entsprechenden Kenngrößen, insbesondere die magnetische Leitfähigkeit bzw. Eigenschaft des Indikatorprofils, die ursprüngliche Seillänge oder die Seilquerschnittsfläche, in einem Referenz-Erfassungsmodus bei neuem oder noch unbeeinträchtigtem Faserseil erfolgen, beispielsweise indem die zuvor beschriebenen Prozeduren mit einem Prüfgewicht bei Inbetriebnahme des Krans durchfahren werden und die hierbei bestimmten Kenngrößen in einem Referenzwertspeicher abgespeichert werden. Im weiteren Betrieb des Krans bzw. Hebezeugs werden die genannten Kenngrößen sodann laufend bzw. zyklisch überwacht und mit den anfänglich abgespeicherten Referenzwerten hierfür verglichen. Zeigt einer oder mehrere der genannten Kenngrößen eine Abweichung gegenüber dem entsprechenden Referenzwert, die ein zulässiges Abweichungsmaß überschreitet, kann die Auswerteeinrichtung ein Ablegesignal bereitstellen. Alternativ oder zusätzlich kann die genannte Auswerteeinrichtung das Ablegesignal auch dann bereitstellen, wenn zwar keine der genannten Kenngrößen einzeln ihren zulässigen Veränderungs- bzw. Abweichungsgrenzwert überschreitet, jedoch die Kenngrößen in Summe betrachtet eine zu große Abweichung von der Summe der Referenzwerte zeigen. Erreichen beispielsweise sämtliche ermittelten Kenngrößen 90 % der zulässigen Abweichung vom Referenzwert, würde jeder Wert einzeln betrachtet noch zulässig sein; dennoch kann die Ablegereife angezeigt werden, da nicht nur einer, sondern sämtliche Kenngrößen beinahe ihre zulässige Veränderungsgrenze erreicht haben.

Die Erfindung wird nachfolgend anhand eines bevorzugten Ausführungsbeispiels und zugehöriger Zeichnungen näher erläutert. In den Zeichnungen zeigen:
- Fig. 1:: eine schematische Darstellung eines erfindungsgemäßen Hebezeugs in Form einer Turmdrehkrans nach einer vorteilhaften Ausführung der Erfindung, dessen Hubseil und/oder dessen Abspannseile für den wippbaren Ausleger als Faserseile ausgebildet sein können,
- Fig. 2:: eine schematische Darstellung eines Turmdrehkrans ähnlich Fig. 1 in einer modifizierten Ausführung, gemäß der das Hubseil nicht über die Spitze eines wippbaren Auslegers, sondern über eine entlang dem Ausleger verfahrbaren Laufkatze abläuft,
- Fig. 3:: eine schematische Darstellung der Erfassungsmittel zur magnetinduktiven Überwachung von Veränderungen eines in das Faserseil eingebetteten Indikatorprofils,
- Fig. 4:: eine schematische Darstellung der Erfassungsmittel zur Erfassung einer Längung des Faserseils, und
- Fig. 5:: eine schematische Darstellung der Erfassungsmittel zur Erfassung von Querschnittsveränderungen des Faserseils.

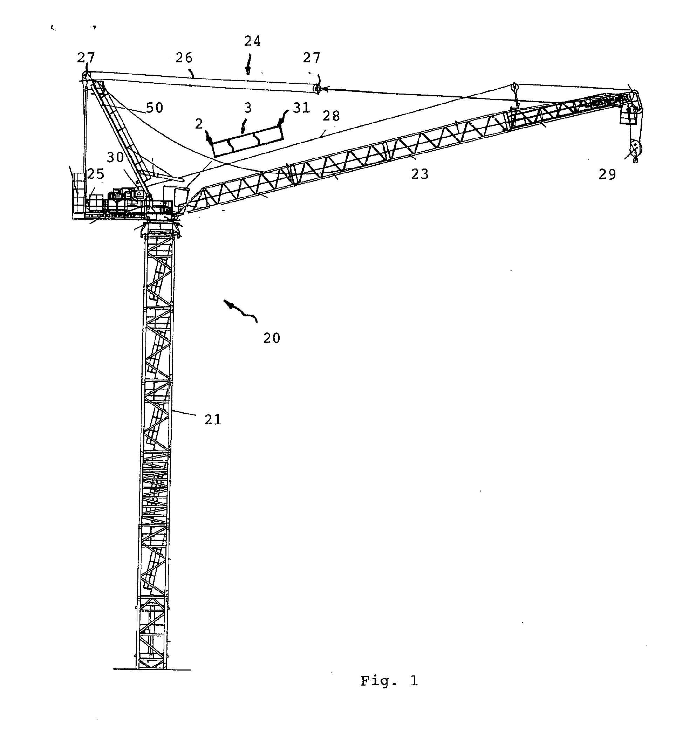

Fig. 1 zeigt beispielhaft für ein Hebezeug nach einer vorteilhaften Ausführung der Erfindung einen Kran in Form eines oben drehenden Turmdrehkrans 20, dessen Turm 21 auf einem Wagen oder einer feststehenden Basis gelagert ist. An dem Turm 21 ist in an sich bekannter Weise ein Ausleger 23 um eine liegende Achse wippbar angelenkt und über eine Abspannverseilung 24 abgespannt. Die genannte Abspannverseilung 24 ist über eine Abspannseilwinde 25 in ihrer Länge veränderbar, so dass der Ausleger 23 in seinem Anstellwinkel verändert werden kann. Hierzu läuft ein Abspannseil 26 auf die genannte Abspannseilwinde 25 auf. Über Umlenkrollen 27 beispielsweise an der gezeigten Abspannstrebe 50 oder einer Turmspitze wird das Abspannseil 26, bzw. die Abspannverseilung 24 an einen Anlenkpunkt am Ausleger 23 in der Nähe der Spitze des Auslegers 23 geführt.

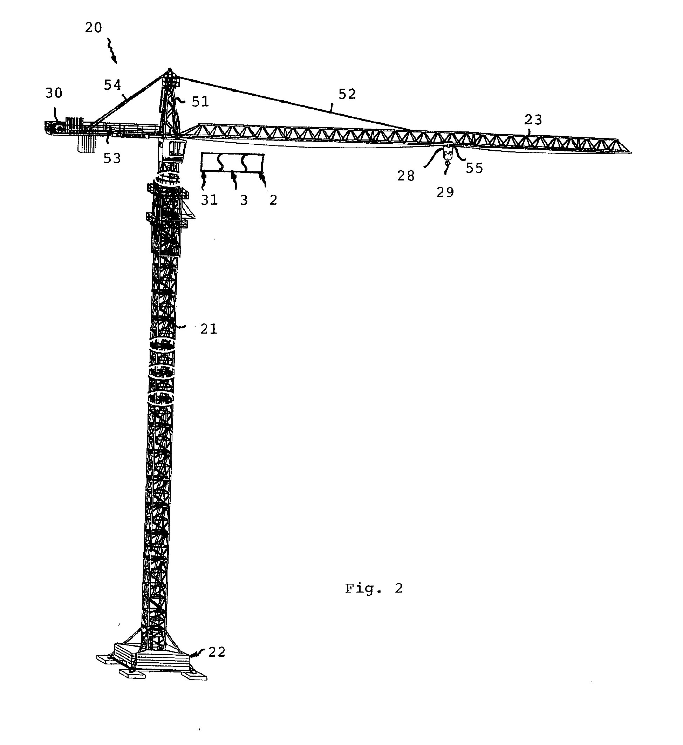

Wie Fig. 2 zeigt, kann der Turmdrehkran 20 natürlich auch mit einem Laufkatzenausleger versehen sein. Der ebenfalls oben drehende Turmdrehkran 20, dessen Turm 21 an der mit Ballast versehenen Basis 22 verankert ist, besitzt ein in der Betriebsstellung liegend, insbesondere horizontal ausgerichteten Ausleger 23, der über Abspann-Zugmittel beispielsweise in Form von Abspannstangen 52 an der Turmspitze 51 angespannt ist, wobei auch der mit Ballast versehene Gegenausleger 53 über Abspann-Zugmittel 54 an der genannten Turmspitze 51 abgespannt ist. An dem vorgenannten Ausleger 23 ist eine Laufkatze 55 verfahrbar gelagert, wobei die genannte Laufkatze 55 beispielsweise mittels eines Laufkatzenseils verfahren werden kann, das über Umlenkrollen an der Auslegerspitze geführt sein kann. Ferner umfasst der Turmdrehkran ein Hubseil 28, das in der gezeichneten Ausführung nach Fig. 1 über Umlenkrollen an der Auslegerspitze von der Spitze des Auslegers herabgelassen werden kann und dort mit einem Kranhaken 29 verbunden ist, oder in der Ausführung nach Fig. 2 über die besagte verfahrbare Laufkatze 55 und dort vorgesehene Umlenkrollen ablaufen und mit dem Kranhaken 29 verbunden sein kann. Das genannte Hubseil 28 läuft in beiden Fällen auf eine Hubwinde 30 auf, die wie die Abspannseilwinde 25 der Ausführung nach Fig. 1 im Bereich des Ballastrahmens oder einem anderen Trägerteil am Gegenausleger 53 angeordnet ist.

Das genannte Hubseil 28 und/oder das Abspannseil 26 können hierbei als Faserseil ausgebildet sein, das aus Kunstfasern wie beispielsweise Aramidfasern oder einem Aramid-/Kohlefasergemisch bestehen kann.

Um für die Ablegereife relevante Kenngrößen des genannten Faserseils überwachen bzw. erfassen zu können, ist eine Erfassungseinrichtung 2 vorgesehen, die am Kran angeordnet sein kann und zusammen mit einer Auswerteeinrichtung 3, die die erfassten Kenngrößen auswertet, mit der elektronischen Kransteuereinheit 31 verbunden oder in diese integriert sein kann.

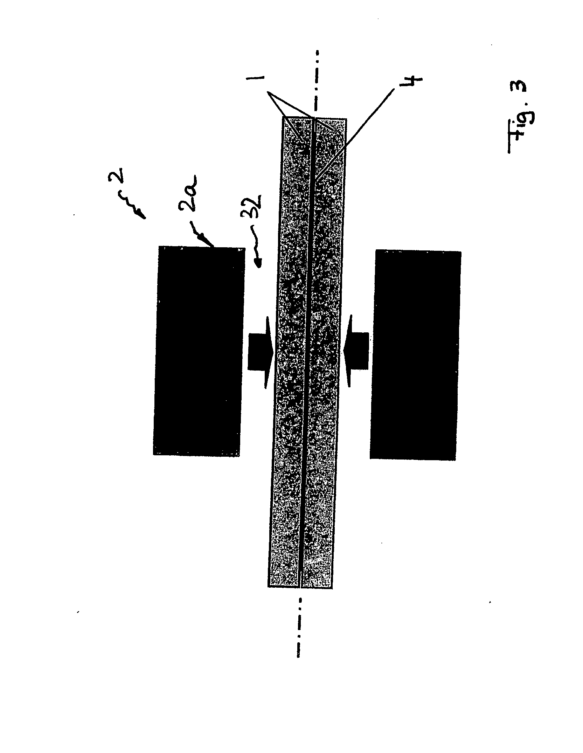

Wie die Figuren 3-5 zeigen, umfasst die genannte Erfassungseinrichtung 2 hierbei vorteilhafterweise verschiedene Erfassungsmittel, um verschiedene Kenngrößen des Faserseils 1 in unterschiedlicher Weise zu erfassen. Gemäß Fig. 3 kann die genannte Erfassungseinrichtung 2 magnetisch arbeitende Erfassungsmittel 2a umfassen, die Veränderungen eines in das Faserseil 1 eingebetteten Indikatorprofils 4 erfassen, das magnetisch leitend bzw. ein Magnetfeld beeinflussend bzw. magnetisierbar ausgebildet ist und mit verseilt sein kann. Beispielsweise kann das genannte Indikatorprofil 4 im Kern in der Litze oder dazwischen angeordnet sein, wobei das genannte Indikatorprofil 4 selbst grundsätzlich beliebige Querschnittsformen besitzen kann, vorteilhafterweise mit einem runden Querschnitt versehen sein kann. Insbesondere kann das genannte Indikatorprofil 4 aus einem metallischen Endlosmaterial wie beispielsweise einem Draht geformt sein, wobei das Indikatorprofil 4 vorteilhafterweise derart beschaffen ist, dass es gegenüber Seilbelastungen, Dehnungen, Zug, Biegung, Torsion, Temperatur und anderen relevanten Eigenschaften weniger widerstandsfähig ausgelegt ist als die Fasern des Faserseils 1 bzw. das Faserseil 1 selbst, so dass das Indikatorprofil 4 versagt, bevor es zu einem Versagen des Faserseils 1 kommt.

Die genannten magnetischen Erfassungsmittel 2a, die beispielsweise einen Magnetfeldsensor umfassen können, erfassen die Veränderungen eines Magnetfelds, das auf das genannte Indikatorprofil 4 einwirkt oder von diesem erzeugt wird. Insbesondere ein Bruch des genannten Indikatorprofils 4 führt hierbei zu Veränderungen des genannten Magnetfelds 32, so dass aus der Erfassung der entsprechenden, charakteristischen Magnetfeldänderung auf einen Bruch des Indikatorprofils 4 und hieraus wiederum auf die Ablegereife des Faserseils 1 geschlossen werden kann.

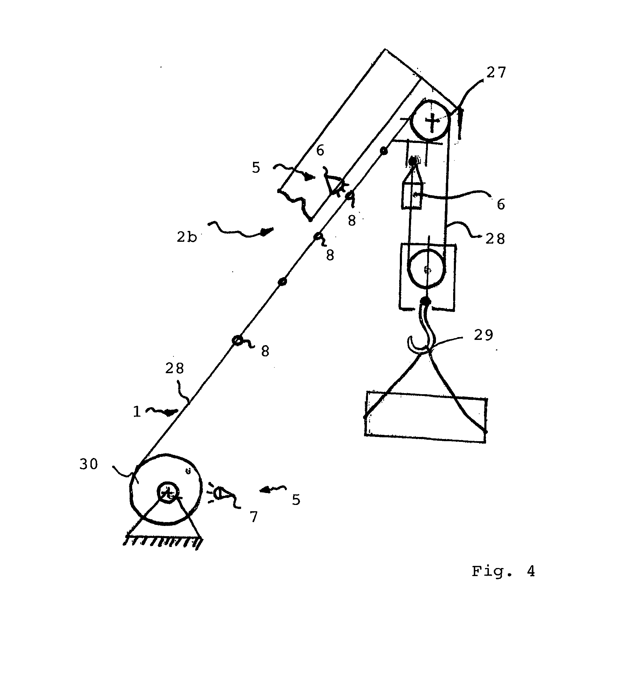

Um bestimmen zu können, in welchem Bereich des Faserseils 1 der Bruch des Indikatorprofils 4 auftritt, kann der Erfassungseinrichtung 2 bzw. deren magnetischen Erfassungsmittel 2a eine Seilwegmessung zugeordnet sein, die durch geeignete Seilwegerfassungsmittel 5 bewerkstelligt wird, beispielsweise dadurch, dass ein der Seilwinde zugeordneter Drehstellungssensor 7, vgl. Fig. 4, die Drehstellung der Seilwinde angibt oder Positionssensoren 6, vgl. Fig. 4, gekennzeichnete Seilabschnitte an einer bestimmten Position erfassen, bei der bzw. denen die genannten magnetischen Erfassungsmittel 2a die Fehlstelle melden. Aus der bekannte Position der Erfassungsmittel 2a kann die Auswerteeinrichtung 3 exakt bestimmten, wo die Fehlstelle ermittelt wurde. Vorteilhafterweise wird aufgrund einer noch bestehenden Restlebensdauer des hochfesten Faserseils der notwendige Zeitraum zum Austausch des Faserseils 1 z.B. am Monitor der Kransteuerung angezeigt. Sollte es nicht zum Tausch in der vorgegebenen Zeit kommen, kann die Kransteuereinheit 31 den Kran zur Sicherheit automatisch stilllegen.

Wie Fig. 4 zeigt, umfasst die vorgenannte Erfassungseinrichtung 2 vorteilhafterweise weiterhin Erfassungsmittel 2b zur Bestimmung einer im Betrieb nach und nach auftretenden Längung des Faserseils 1. Hierbei kann mit dem Faserseil 1 eine bestimmte Position angefahren werden, beispielsweise durch direktes Anfahren des oberen Abschaltpunktes, bei dem der Lasthaken 29 die höchstmögliche Position erreicht hat und was beispielsweise durch einen Endschalter oder einen anderen Positionssensor 6 erfasst werden kann. Meldet der genannte Positionssensor 6 den Erfassungsmitteln 2b, dass die vorbestimmte Seilposition erreicht ist, wird durch einen Seilwindenstellungssensor 7 die Stellung der Seilwinde erfasst bzw. bestimmt. Diese Messung wird zunächst bei der ersten Inbetriebnahme des Krans durchgeführt. Stellt sich bei späteren Messungen eine andere Seilwindenposition ein, wenn die vorbestimmte Seilposition angefahren wird, ist die Abweichung der Seiltrommelstellung für das Anfahren desselben Seilpunktes ein Maß für die aufgetretene Längung des Faserseils 1. Vorteilhafterweise werden die Prüfzyklen mit einer vorbestimmten Last am Lasthaken 29, beispielsweise einer bekannte Prüflast durchgeführt, so dass keine variierenden Randbedingungen die Messgenauigkeit beeinflussen.

Bei dieser Methode der Erfassung der Seillängung durch Messung der zunehmenden Trommelumdrehung bis zum Abschaltpunkt ist zu beachten, dass es sich um einen Durchschnittswert der Seillängung handelt. Die Seildehnung ist abhängig von der Belastung und der Dauer der Belastung. Wird eine Last bewegt z.B. "Heben", so hat der Seilbereich, der nicht auf die Seiltrommel gespult immer die volle und längste Belastung, bis die Last wieder abgesetzt wird. Im Seilbereich, der auf die Trommel gespult wird, nimmt der Seilzug kontinuierlich ab und somit auch die Beanspruchung auf Dehnung. Somit wird die Dehnung des Seiles außerhalb der Seiltrommel ca. konstant verlaufen und immer die maximale Beanspruchung bekommen. Beim Seil, das auf die Trommel gespult wird, nimmt die bestehende Zuglast kontinuierlich ab, da die Seilbelastung auf Zug nach einigen Windungen auf ca. null abnimmt. Die Grenze der zulässigen Längung kann bei dieser Methode mit einem Dehnungsverteilfaktor in Bezug auf die gesamte Seillänge ermittelt werden, um eine ausreichende Sicherheit für den Zeitpunkt der Ablegereife des Faserseils 1 zu erhalten.

Eine weitere Methode der Seillängungsprüfung bezüglich Ablegereife beruht auf Signalgebern 8 bzw. Indikatoren, die aktiv oder inaktiv Signale abgeben. Diese Indikatoren sind im Seil mit ca. gleichen Abständen fest integriert. Eine z.B. elektrisch-elektronische Messeinrichtung beispielsweise in Form eines Positionssensors 6 erfasst den Punkt des Indikators und misst den Längenabstand bis zum nächsten Indikator bei konstanter Seilgeschwindigkeit. Somit kann die Seillänge auf beliebige Messpunkte aufgeteilt werden und man erhält mit dieser Methode eine Auswertung über den Dehnungsverlauf des Seiles über die gesamte Seillänge und erkennt mit einer Messeinrichtung, in welchem Seilbereich der Grenzwert erreicht wurde und das Seil wird ausgetauscht, oder, wenn möglich, um den Ablegebereich, d.h. den überdehnten Seilbereich gekürzt.

Die Messeinrichtung wird bei der ersten Inbetriebnahme eingestellt. Es wird eine vorgegebene Hubbewegung mit Modus z.B. "Seilprüfung" durchgeführt. Dabei wird mit konstanter Hubgeschwindigkeit aus der untersten Position des Lasthakens 29 in die oberste Position gefahren. Bei diesem Ablauf werden die Abstandslängen der genannten mehreren Indikatoren 8 erfasst und gespeichert. Zu einem späteren Zeitpunkt nach einer entsprechender Einsatzdauer wird der Vorgang wiederholt und die Längendifferenz zur ersten Messung errechnet und angezeigt. Die Messeinrichtung gibt die Werte an die Kransteuerung und das Speichermodul weiter, von der Kransteuerung ist eine Weitergabe per DFÜ möglich bzw. wird der Kranführer über den Zustand des Seiles am Kranmonitor unterrichtet. Beim Erreichen von unzulässigen Dehnungen wird ein Sicherheitsmodus aktiv und bei Nichtbeachtung erfolgt nach einer zulässigen Resteinsatzzeit die Warnung und Abschaltung der Anlage. Aus Sicherheitsgründen kann die Anlage nicht mehr in Betrieb genommen werden, wenn diese außer Betrieb genommen wurde. Die Stillstandsursache wird auch am Monitor angezeigt und kann auch über Datenfernübertragung abgerufen werden.

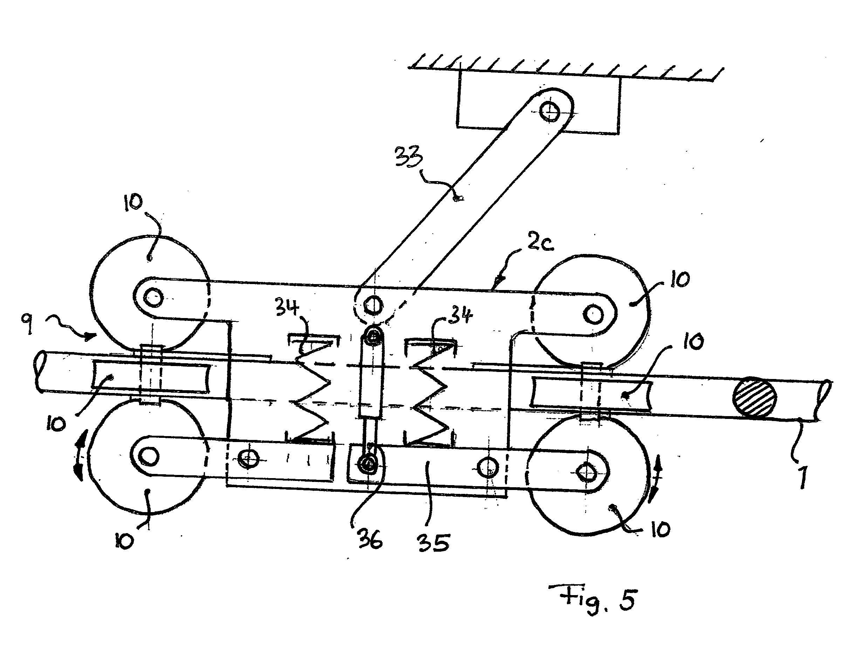

Wie Fig. 5 zeigt, kann die Erfassungseinrichtung 4 vorteilhafterweise auch Erfassungsmittel 2c zur Bestimmung von Veränderungen des Seilquerschnitts und/oder der Querdrucksteifigkeit des Faserseils 1 umfassen. Die genannten Erfassungsmittel 2c erfassen hierzu vorteilhafterweise in mindestens zwei Ebenen, die vorteilhafterweise zueinander senkrecht stehen können, den Seildurchmesser bzw. die Querdrucksteifigkeit, um auch bei an sich noch unschädlichen Veränderungen der Seilquerschnittsform die Seilquerschnittsfläche aus den mehreren Seildurchmessern bestimmen zu können. Dies hat den Hintergrund, dass hochfeste Faserseile 1 unter Querbelastungen wie sie an den Umlenkrollen 27 oder an den Seilwinden 25 bzw. 30 auftreten, zu einer Ovalisierung im Querschnitt neigt, die an sich noch keine Beeinträchtigung der Seilfestigkeit mit sich bringt. Kritisch wird es indes, wenn die Seilquerschnittsfläche abnimmt.

Bei der Ausführung nach Fig. 5 werden hierfür die Seildurchmesser in zueinander senkrechten Ebenen mittels Klemmmittelpaaren in Form von Seilrollen 10 mechanisch abgetastet, die von gegenüberliegenden Seiten her gegen die Oberfläche des Faserseils 1 gedrückt werden, so dass die lichte Weite zwischen den Klemmmitteln in Form der Seilrollen 10 ein Maß für den entsprechenden Seildurchmesser ist.

Wie Fig. 5 zeigt, sind die Erfassungsmittel 2c insgesamt quer zur Seillängsrichtung beweglich gelagert, so dass Querbewegungen des Faserseils 1 keine Auswirkungen auf das Messergebnis haben. In der gezeichneten Ausführung ist die gesamte Vorrichtung hierbei über einen Schwenkrahmen bzw. eine Hebelanlenkung 33 querbeweglich aufgehängt, vgl. Fig. 5.

Die Messeinrichtung besitzt vorteilhafterweise in einer Ebene mindestens zwei Rollen im Vorderen und zwei Rollen im hinteren Bereich, davon jeweils die untere Rolle über Federn 34 das Seil 1 leicht klemmt und somit den Seildurchmesser erfasst. Eine dieser unteren gefederten Rollen 10 besitzt eine Drehachse und einen Hebel 35 über den der gemessene Seildurchmesser auf einen Wegsensor 36 übertragen und somit ausgewertet wird. Die Messeinheit besitzt weitere seitliche Führungsrollen zum Seil, so dass die Messeinheit über das Seil geführt wird und eventuelle Seilschwingungen auf die Messwerte keinen Einfluss haben. Die Messeinheit ist über einen Hebel gelenkig zum Stahlbau des Kranes aufgehängt, um Bewegungen auszugleichen. Die Seilmessung erfolgt vorteilhafterweise mindestens über zwei Ebenen des Seils um 90° versetzt, so dass der Seildurchmesser über vier Bereiche geprüft wird. Eine weitere versetzte Anordnung, z.B. für sechs Bereiche, ist möglich. Die Messung über 2 - 4 - 6 usw. Bereiche kann in einer Messeinheit konstruktiv vorgesehen werden, oder durch Anordnung mehrerer Messeinheiten.

Eine weitere Möglichkeit besteht durch Einsatz optischer Prüfgeräte, die eine Seildurchmesserveränderung bezogen auf den Umfang erkennen und auswerten. Bei Über- oder Unterschreitung der zulässigen Durchmesserabweichung wird ein Warnsignal gegeben und die Position gespeichert über den Trommeldrehzahlsensor 7.

Um mit der zuvor gezeigten Messeinrichtung gemäß Fig. 5 auch die Querdrucksteifigkeit präzise bestimmen zu können, kann in Weiterbildung der Erfindung den Klemmbacken bzw. Klemmrollen 10 eine Stellvorrichtung zugeordnet sein, mittels derer eine variable und/oder ausreichend hohe Querkraftbeaufschlagung des Seils erzeugt werden kann, d.h. die Rollen 10 mit ausreichender Kraft quer gegen das Seil gedrückt werden können. Vorteilhafterweise kann hierbei die jeweils beaufschlagte Querkraft durch eine geeignete Kraftmesseinrichtung gemessen werden. Durch den Wegsensor 36 wird die sich einstellende Verformung des Seils 1 gemessen, wobei die eingangs erläuterten Veränderungen von Stellkraft und/oder Querverformung in einem oder mehreren Messzyklen bewerkstelligt werden können.

Bei Nichtbeachtung des Warnsignals erfolgt vorteilhafterweise nach einer zulässigen Resteinsatzzeit die Warnung und Abschaltung der Anlage. Aus Sicherheitsgründen kann die Anlage nicht mehr in Betrieb genommen werden, wenn diese außer Betrieb genommen wurde. Die Stillstandsursache wird auch am Monitor angezeigt und kann auch über Datenfernübertragung abgerufen werden.

Ferner kann die genannte Erfassungseinrichtung 2 vorteilhafterweise auch Erfassungsmittel 2d zur Erfassung des auf das jeweilige Faserseil 1 einwirkenden Lastkollektivs aufweisen, wobei hier vorteilhafterweise zumindest die auf das Seil einwirkende Zuglast und die Anzahl der Biegewechsel, vorteilhafterweise aber auch andere die Dauerfestigkeit beeinflussenden Parameter wie mehrlagige Spulung, Umwelteinflüsse, Temperatur, Querbelastungen und anderes erfasst werden kann.

Zur Ermittlung der genannten Parameter umfassen die genannten Erfassungsmittel 2d entsprechende Sensoren, deren Signale in der genannten Auswerteeinheit 3 erfolgt. Insbesondere kann ein Lastmesssensor die laufende Belastung über die Betriebszeit des Seils erfassen. Vorteilhafterweise kann ferner ein Drehwegsensor auf der jeweiligen Windentrommel die Seillänge messen, die beansprucht wird. In der Summe kann hieraus ein Lastkollektiv beispielsweise in Form einer Wöhlerkurve bestimmt werden, das mit einem vorgegebenen maximalen Lastkollektiv für das Faserseil 1 verglichen werden kann. Wird die Anzahl des maximal zulässigen Lastkollektivs, also eine bestimmte Anzahl von Biegewechseln unter Einfluss einer bestimmten Last und/oder bestimmte Lastspitzen, erreicht, kann eine Warnung und/oder eine Vorgabe, in welcher Zeit der Seilwechsel erfolgen muss, vorgenommen werden.

## Patentansprüche

1. Vorrichtung zur Erkennung der Ablegereife eines hochfesten Faserseils (1) beim Einsatz an Hebezeugen, insbesondere Kran, mit einer Erfassungseinrichtung (2) zur Erfassung zumindest einer Seilkenngröße sowie einer Auswerteeinrichtung (3) zur Auswertung der Seilkenngröße sowie Bereitstellung eines Ablegesignals in Abhängigkeit der Seilkenngrößenauswertung, **dadurch gekennzeichnet, dass** die Erfassungseinrichtung (2) Erfassungsmittel (2b) zur Erfassung einer Längung des Faserseils (1) aufweist und die Auswerteeinrichtung (3) die bestimmte Längung mit einer zulässigen maximalen Längung abgleicht und bei Überschreiten der zulässigen maximalen Längung das Ablegesignal bereitstellt.

2. Vorrichtung nach dem vorhergehenden Anspruch, wobei die Erfassungsmittel (2b) einen Positionssensor (6) zur Erfassung eines vorbestimmten Seilpunkts an einer vorbestimmten Position, insbesondere eines oberen Abschaltpunkts für den Lasthaken, sowie einen Seilwindenstellungssensor (7) zur Erfassung der beim Anfahren der vorbestimmten Seilpunktposition sich einstellenden Windenstellung aufweisen und die Auswerteeinrichtung (3) die Veränderung der sich einstellenden Windenstellung überwacht und dann, wenn die Veränderung der Windenstellung ein vorbestimmtes Maß überschreitet, das Ablegesignal bereitstellt.

3. Vorrichtung nach einem der beiden vorhergehenden Ansprüche, wobei die Erfassungsmittel (2b) zur Erfassung der Längung im Faserseil (1) über dessen Länge verteilt mehrere Signalgeber (8), beispielsweise in Form von Markierungen, Transpondern, Signalreflektoren oder dergleichen aufweisen und Bestimmungsmittel zur Bestimmung des Abstands zwischen jeweils zwei Signalgebern (8) aufweisen und die Auswerteeinrichtung (3) die Veränderung des bestimmten Abstands zwischen jeweils zwei Signalgebern (8) auswertet und bei Überschreiten einer vorbestimmten Abstandsänderung das Ablegesignal bereitstellt.

4. Vorrichtung nach einem der vorhergehenden Ansprüche, wobei die Erfassungseinrichtung (2) Querdrucksteifigkeits- und/oder Querschnitts-Bestimmungsmittel (2c) zur Bestimmung der Querdrucksteifigkeit und/oder Querschnittsfläche und/oder -form des Seils aufweist und die Auswerteeinheit (3) das Ablegesignal in Abhängigkeit der bestimmten Querdrucksteifigkeit und/oder Querschnittsfläche und/oder -form des Seils auswertet.

5. Vorrichtung nach dem vorhergehenden Anspruch, wobei die Auswerteeinheit (3) die Querdrucksteifigkeit und/oder Querschnittsfläche und/oder -form des Seils auf Veränderungen überwacht sowie bei Überschreiten einer vorbestimmten Veränderung das Ablegesignal bereitstellt, und/oder die bestimmte Querdrucksteifigkeit und/oder Querschnittsfläche mit einem vorbestimmten Grenzwert für die Querdrucksteifigkeit und/oder Querschnittsfläche vergleicht und bei Überschreiten/Unterschreiten des Grenzwerts das Ablegesignal bereitstellt.

6. Vorrichtung nach dem vorhergehenden Anspruch, wobei die Querdrucksteifigkeits- und/oder Querschnittsbestimmungsmittel (2c) als Erfassungsmittel (9) zumindest ein elastisch vorspannbares Klemmmittelpaar, vorzugsweise gegen das Faserseil (1) drückbare Klemmbacken oder Seilrollen (10), die quer zur Seillängsrichtung beweglich aufgehängt sind, und Abstandsmessmittel (36) zur Messung des Abstands des Klemmmittelpaars voneinander aufweisen.

7. Vorrichtung nach einem der vorhergehenden Ansprüche, wobei die Querdrucksteifigkeits-Bestimmungsmittel (2c) derart ausgebildet sind, dass das Seil (1) bei der Querdrucksteifigkeitsbestimmung einer vorbestimmten Zugbelastung unterwerfbar ist, wobei das unter der vorbestimmten Zugbelastung stehende Seil (1) mit einer quer zur Seillängsrichtung wirkenden Querkraft beaufschlagbar und die bei/durch die Querkraftbeaufschlagung entstehende Querschnitts- und/oder Durchmesserverformung des Seils (1) bestimmbar ist.

8. Vorrichtung nach einem der vorhergehenden Ansprüche, wobei die Auswerteeinheit (3) das Ablegesignal bei Auftreten/Überschreiten einer vorbestimmten Zunahme der Querdrucksteifigkeit und/oder bei Erreichen/Überschreiten eines vorbestimmten Querdrucksteifigkeitsgrenzwerts bereitstellt.

9. Vorrichtung nach einem der vorhergehenden Ansprüche, wobei die Querdrucksteifigkeits- und/oder Querschnittsbestimmungsmittel (2c) Erfassungsmittel (9) zur Erfassung der Querdrucksteifigkeit und/oder des Seildurchmessers in zumindest zwei verschiedenen Ebenen umfassen und die Querdrucksteifigkeit und/oder Seilquerschnittsfläche aus den zumindest zwei bestimmten Querdrucksteifigkeiten und/oder Seildurchmessern bestimmt.

10. Vorrichtung nach einem der vorhergehenden Ansprüche, wobei die Erfassungseinrichtung (2) Erfassungsmittel (2a) zur Erfassung einer Veränderung eines Indikatorprofils (4), das in das Faserseil (1) eingebettet ist und aus einem von den Seilfasern verschiedenen Material besteht, aufweist.

11. Vorrichtung nach dem vorhergehenden Anspruch, wobei die Erfassungsmittel (2a) magnetisch arbeitend ausgebildet sind, insbesondere einen Magnetfeldsensor umfassen und das Indikatorprofil (4) aus einem Magnetfeld beinflussenden und/oder magnetisch leitenden und/oder magnetisierbaren Material, vorzugsweise einem metallischen Endlosprofil, besteht und/oder wobei das Indikatorprofil (4) hinsichtlich seiner Widerstandsfähigkeit gegen Seilspannungen, Dehnung, Zug, Biegung, Torsion, UV-Strahlung, Wasseraufnahme und/oder Temperatur schwächer als das Faserseil (1) und dessen Seilfasern ausgebildet ist derart, dass das Indikatorprofil (4) versagt, insbesondere bricht oder reißt, bevor ein Versagen des Faserseils (1) eintritt.

12. Vorrichtung nach einem der vorhergehenden Ansprüche, wobei die Erfassungseinrichtung (2) mehrere, verschieden ausgebildete Erfassungsmittel (2a, 2b, 2c, 2d, 2n) zur magnetischen, mechanischen, optischen und/oder elektronischen Erfassung mehrerer, verschiedener Seilkenngrößen umfasst, die von der genannten Auswerteeinrichtung (3) einzeln und/oder in Kombination miteinander zur Erkennung der Ablegereife auswertbar sind.

13. Vorrichtung nach dem vorhergehenden Anspruch, wobei die Auswerteeinrichtung (3) ein Ablegesignal dann, wenn zumindest eine der erfassten Seilkenngrößen oder deren Veränderung einen zugehörigen Grenzwert überschreitet/unterschreitet, sowie dann, wenn eine aus allen oder einigen der erfassten Seilkenngrößen abgeleitete, mittelbare Summenkenngröße oder deren Veränderung einen zugehörigen Grenzwert überschreitet/unterschreitet, abgibt.

14. Vorrichtung nach einem der vorhergehenden Ansprüche, wobei die Erfassungseinrichtung (2) einen Lastkollektivzähler (2d) zur Erfassung des auf das Faserseil (1) einwirkenden Lastkollektivs umfassend die Seilzugbelastung und die Biegewechselanzahl besitzt und/oder wobei der Erfassungseinrichtung (2) Seilweg- und/oder -positionserfassungsmittel (5) zur Bestimmung der Seilabschnitte, in denen Veränderungen der erfassten Seilkenngröße auftreten, zugeordnet sind und die Auswerteeinrichtung (3) zusammen mit dem Ablegesignal ein Seilabschnittsignal, das angibt, welcher Seilabschnitt ablegereif ist, bereitstellt.

15. Kran, insbesondere Turmdrehkran, Mobilkran, Hafenmobilkran, Schiffskran oder Fahrzeug-Auslegerkran, mit einer Vorrichtung nach einem der vorhergehenden Ansprüche.

## Claims

1. An apparatus for recognizing the discard state of a high-strength fiber rope (1) in use at lifting gear, in particular a crane, comprising a detection device (2) for detecting at least one rope parameter as well as comprising an evaluation device (3) for evaluating the rope parameter and for providing a discard signal in dependence on the rope parameter evaluation, **characterized in that** the detection device (2) comprises detection means (2b) for detecting a lengthening of the fiber rope (1) and the evaluation device (3) compares the determined lengthening with a permitted maximum lengthening and provides the discard signal on an exceeding of the permitted maximum lengthening.

2. An apparatus in accordance with the preceding claim, wherein the detection means (2b) have a position sensor (6) for detecting a predefined rope point at a predefined position, in particular an upper switch-off point for the load hook, as well as a rope winch position sensor (7) for detecting the winch position adopted on a traveling to the predefined rope point position and the evaluation device (3) monitors the change in the adopted winch position and provides the discard signal when the change in the winch position exceeds a predefined amount.

3. An apparatus in accordance with the one of the two preceding claims, wherein the detection means (2b) have a plurality of signalers (8), for example in the form of marks, transponders, signal reflectors or the like distributed over the length of the fiber rope (1) for detecting the lengthening in the fiber rope and have determination means for determining the distance between two respective signalers (8) and the evaluation device (3) evaluates the change in the determined distance between two respective signalers (8) and provides the discard signal on an exceeding of a predefined distance change.

4. An apparatus in accordance with one of the preceding claims, wherein the detection device (2) has transverse compressive stiffness determination means and/or cross-section determination means (2c) for determining the transverse compressive stiffness and/or the cross-section area and/or cross-section shape of the rope and the evaluation device (2) evaluates the discard signal in dependence on the determined transverse compressive stiffness and/or cross-section area and/or cross-section shape of the rope.

5. An apparatus in accordance with the preceding claim, wherein the evaluation device (3) monitors the transverse compressive stiffness and/or cross-section area and/or cross-section shape of the rope for changes and provides the discard signal on an exceeding of the predefined change and/or compares the determined transverse compressive stiffness and/or cross-section area with a predefined limit value for the transverse compressive stiffness and/or cross-section area and provides the discard signal on an exceeding of/falling below the limit value.

6. An apparatus in accordance with the preceding claim, wherein the transverse compressive stiffness determination means and/or cross-section determination means (2c) have as detection means (9) at least one elastically preloadable clamping means pair, preferably clamping jaws or rope rollers (10) which can be pressed against the fiber rope (1) and which are movably suspended transversely to the longitudinal direction of the rope and distance measurement means (36) for measuring the distance of the clamping means pair from one another.

7. An apparatus in accordance with one of the preceding claims, wherein the transverse compressive stiffness determination means (2c) are configured such that the rope (1) can be subjected to a predefined tensile load in the transverse compressive stiffness determination, wherein the rope (1) under the predefined tensile load can be acted on by a transverse force acting transversely to the longitudinal direction of the rope and the cross-sectional deformation and/or diameter deformation of the rope (1) arising in/by the transverse force action can be determined.

8. An apparatus in accordance with one of the preceding claims, wherein the evaluation device (3) provides the discard signal on an occurrence/exceeding of a predefined increase in the transverse compressive stiffness and/or on reaching/exceeding a predefined transverse compressive stiffness limit value.

9. An apparatus in accordance with one of the preceding claims, wherein the transverse compressive stiffness determination means and/or cross-section determination means (2c) comprise detection means (9) for detecting the transverse compressive stiffness and/or the rope diameter in at least two different planes and determines the transverse compressive stiffness and/or the rope cross-sectional area from the at least two determined transverse compressive stiffnesses and/or rope diameters.

10. An apparatus in accordance with one of the preceding claims, wherein the detection device (2) comprises detection means (2a) for detecting a change in an indicator section (4) which is embedded in the fiber rope (1) and comprises a material different from the rope fibers.

11. An apparatus in accordance with the preceding claim, wherein the detection means (2a) are configured as working magnetically, in particular include a magnetic field sensor and the indicator section (4) comprises a material, preferably a metallic continuous section, influencing a magnetic field and/or magnetically conductive and/or magnetizable, and/or wherein the indicator section (4) is configured weaker than the fiber rope (1) and its rope fibers with respect to its resistance capability to rope strains, stretch, tension, bending, torsion, UV radiation, water absorption and/or temperature such that the indicator section (4) fails, in particular breaks or tears, before a failure of the fiber rope (1) occurs.

12. An apparatus in accordance with one of the preceding claims, wherein the detection device (2) comprises a plurality of differently configured detection means (2a, 2b, 2c, 2d, 2n) for the magnetic, mechanical, optical and/or electronic detection of a plurality of different rope parameters which can be evaluated by the named evaluation device (3) individually and/or in combination with one another for recognizing the discard state.

13. An apparatus in accordance with the preceding claim, wherein the evaluation device (3) outputs a discard signal when at least one of the detected rope parameters or their change exceeds/falls below an associated limit value as well as when an indirect sum parameter or its change derived from all or some of the detected rope parameters exceeds/falls below an associated limit value.

14. An apparatus in accordance with one of the preceding claims, wherein the detection device (2) has a load spectrum counter (2d) for detecting the load spectrum comprising the rope tensile stress and the number of bending cycles acting on the fiber rope (1) and/or wherein the detection device (2) has rope path detection means and/or rope position detection means (5) for determining the rope sections in which changes of the detected rope parameter occur associated with it and the evaluation device (3), together with the discard signal, provides a rope section signal which indicates which rope section is in a discard state.

15. A crane, in particular a revolving tower crane, a mobile crane, a harbor mobile crane, a ship's crane or a vehicle boom crane, having an apparatus in accordance with one of the preceding claims.

## Revendications

1. Dispositif de détection de l'état d'usure d'un câble en fibre (1) haute résistance pour l'utilisation sur des engins de levage, en particulier une grue, comprenant un dispositif de détection (2) destiné à détecter au moins une caractéristique de câble, ainsi qu'un dispositif d'évaluation (3) destiné à évaluer la caractéristique de câble ainsi qu'à produire un signal de dépose en fonction de l'évaluation de la caractéristique de câble, **caractérisé en ce que** le dispositif de détection (2) comporte des moyens de détection (2b) destinés à détecter un allongement du câble en fibre (1) et le dispositif d'évaluation (3) compare l'allongement déterminé avec un allongement maximal admissible et produit le signal de dépose en cas de dépassement de l'allongement maximal admissible.

2. Dispositif selon la revendication précédente, dans lequel les moyens de détection (2b) comportent un capteur de position (6) destiné à détecter un point du câble prédéterminé à une position prédéterminée, en particulier un point de coupure supérieur pour le crochet de levage, ainsi qu'un capteur de position de treuil (7) destiné à détecter le positionnement du treuil lors du déplacement jusqu'à la position du point du câble prédéterminée, et le dispositif d'évaluation (3) surveille la modification du positionnement du treuil et, lorsque la modification de la position du treuil dépasse une grandeur prédéterminée, produit alors le signal de dépose.

3. Dispositif selon l'une des deux revendications précédentes, dans lequel les moyens de détection (2b) destinés à détecter l'allongement dans le câble en fibre (1) comportent, distribués sur sa longueur, plusieurs générateurs de signaux (8), par exemple sous la forme de repères, de transpondeurs, de réflecteurs de signaux ou similaires et comportent des moyens de détermination destinés à déterminer la distance entre deux générateurs de signaux (8) respectifs et le dispositif d'évaluation (3) évalue la modification de la distance déterminée entre deux générateurs de signaux (8) respectifs et produit le signal de dépose en cas de dépassement d'une modification de distance prédéterminée.

4. Dispositif selon l'une des revendications précédentes, dans lequel le dispositif de détection (2) comporte des moyens de détermination de la résistance à la pression transversale et/ou de la section transversale (2c) destinés à déterminer la résistance à la pression transversale et/ou la surface et/ou la forme de section transversale du câble et l'unité d'évaluation (3) évalue le signal de dépose en fonction de la résistance à la pression transversale et/ou de la surface et/ou la forme de section transversale déterminées du câble.

5. Dispositif selon la revendication précédente, dans lequel l'unité d'évaluation (3) surveille les modifications de la résistance à la pression transversale et/ou de la surface et/ou la forme de section transversale du câble et produit le signal de dépose en cas de dépassement d'une modification prédéterminée, et/ou compare la résistance à la pression transversale et/ou la surface de section transversale déterminées avec une valeur limite prédéterminée pour la résistance à la pression transversale et/ou la surface de section transversale et produit le signal de dépose en cas de dépassement par le haut / par le bas de la valeur limite.

6. Dispositif selon la revendication précédente, dans lequel les moyens de détermination de la résistance à la pression transversale et/ou de la section transversale (2c) comportent en tant que moyens de détection (9) au moins une paire de moyens de serrage pouvant être précontraints élastiquement, de préférence des mâchoires de serrage pouvant être pressées contre le câble en fibre (1) ou des poulies (10) qui sont accrochées mobiles transversalement à la direction longitudinale du câble, et des moyens de mesure de distance (36) destinés à mesurer la distance entre les moyens de serrage de la paire de moyens de serrage.

7. Dispositif selon l'une des revendications précédentes, dans lequel les moyens de détermination de la résistance à la pression transversale (2c) sont réalisés de telle sorte que le câble (1) peut être soumis à une contrainte de traction prédéterminée lors de la détermination de la résistance à la pression transversale, le câble (1) se trouvant sous la contrainte de traction prédéterminée pouvant être soumis à l'action d'une force transversale agissant transversalement à la direction longitudinale du câble et la déformation de la section transversale et/ou du diamètre du câble (1) se produisant lors / en raison de l'action de la force transversale pouvant être déterminée.

8. Dispositif selon l'une des revendications précédentes, dans lequel l'unité d'évaluation (3) produit le signal de dépose lorsqu'une croissance prédéterminée de la résistance à la pression transversale se produit / est dépassée et/ou lorsqu'une valeur limite de résistance à la pression transversale prédéterminée est atteinte/dépassée.

9. Dispositif selon l'une des revendications précédentes, dans lequel les moyens de détermination de la résistance à la pression transversale et/ou de la section transversale (2c) comprennent des moyens de détection (9) destinés à détecter la résistance à la pression transversale et/ou le diamètre du câble à au moins deux niveaux différents et déterminent la résistance à la pression transversale et/ou la surface de section transversale du câble à partir des au moins deux résistances à la pression transversale et/ou diamètres de câble déterminés.

10. Dispositif selon l'une des revendications précédentes, dans lequel le dispositif de détection (2) comporte des moyens de détection (2a) destinés à détecter une modification d'un profilé indicateur (4) qui est encastré dans le câble en fibre (1) et qui est composé d'un matériau différent des fibres du câble.

11. Dispositif selon la revendication précédente, dans lequel les moyens de détection (2a) sont réalisés à fonctionnement magnétique, comprennent en particulier un capteur de champ magnétique et le profilé indicateur (4) est composé d'un matériau influençant le champ magnétique et/ou conducteur magnétique et/ou aimantable, de préférence un profilé métallique sans fin et/ou dans lequel le profilé indicateur (4) est conçu plus faible que le câble en fibre (1) et que ses fibres de câble en ce qui concerne sa résistance aux tensions de câble, à l'allongement, à la traction, à la flexion, à la torsion, au rayonnement UV, à l'absorption d'eau et/ou à la température, de sorte que le profilé indicateur (4) cède, en particulier rompt ou se déchire, avant qu'une défaillance du câble en fibre (1) ne se produise.

12. Dispositif selon l'une des revendications précédentes, dans lequel le dispositif de détection (2) comprend plusieurs moyens de détection (2a, 2b, 2c, 2d, 2n) réalisés différemment, destinés à la détection magnétique, mécanique, optique et/ou électronique de plusieurs caractéristiques de câble différentes, qui peuvent être évaluées par ledit dispositif d'évaluation (3) individuellement et/ou en combinaison les unes avec les autres afin de détecter l'état d'usure.

13. Dispositif selon la revendication précédente, dans lequel le dispositif d'évaluation (3) émet un signal de dépose quand au moins une ou la modification de l'une des caractéristiques de câble détectées devient inférieure/supérieure à une valeur limite associée, ainsi que quand une ou la modification d'une caractéristique cumulée indirecte dérivée à partir de toutes ou de quelques-unes des caractéristiques de câble détectées devient inférieure/supérieure à une valeur limite associée.

14. Dispositif selon l'une des revendications précédentes, dans lequel le dispositif de détection (2) possède un compteur d'ensemble des charges (2d) destiné à détecter l'ensemble des charges agissant sur le câble en fibre (1) comprenant la contrainte de traction du câble et le nombre de flexions alternées et/ou dans lequel des moyens de détection de parcours et/ou de position du câble (5) destinés à déterminer les portions de câble dans lesquelles des modifications de la caractéristique de câble détectée se produisent sont associés au dispositif de détection (2) et le dispositif d'évaluation (3) produit avec le signal de dépose un signal de portion de câble qui indique quelle portion de câble a atteint l'état d'usure.

15. Grue, en particulier grue-tour, grue mobile, grue mobile portuaire, grue de navire ou grue à flèche pour véhicule, comprenant un dispositif selon l'une des revendications précédentes.
